# EUROPEAN PATENT APPLICATION

(11) **EP 4 166 157 A1**
(43) Date of publication of application: **19.04.2023**
(21) Application number: 20940637.0
(22) Date of filing: 16.06.2020
(51) Int. Cl.: A61K 38/44, A61K 9/00, A61P 43/00

(54) **COMPOSITION FOR TREATMENT OF GLUTARIC ACIDURIA AND ADMINISTRATION METHOD THEREFOR**

(71) Applicant: Novel Pharma Inc., Seoul 06164 (KR)
(72) Inventor: JIN, Dong Kyu, Seoul 06293 (KR)
(74) Representative: Heubeck, Christian
(86) International application number: PCT/KR2020/007774
(87) International publication number: WO 2021/256579

(57) **Abstract**

The present disclosure pertains to a composition for treatment of glutaric aciduria and an administration method therefor and, specifically, to a pharmaceutical composition comprising recombinant human glutaryl-CoA dehydrogenase (rhGCDH) for treatment of glutaric aciduria and a method for treating glutaric aciduria, the method comprising a step of administering the pharmaceutical composition. Provided according to an aspect of the present disclosure are a pharmaceutical composition comprising recombinant human glutaryl-CoA dehydrogenase (rhGCDH) for treating glutaric aciduria and a novel method for treating glutaric aciduria through *in vivo* administration of the pharmaceutical composition (intravenously and subcutaneously; i.v. and s.c.), whereby an excellent effect is brought about through *in vivo* administration remarkably easier than intraventricular administration, with the expectation of more effectively regulating and treating glutaric aciduria.

## Description

### TECHNICAL FIELD

The present disclosure relates to a composition for treatment of glutaric aciduria and an administration method thereof and, specifically, to a pharmaceutical composition containing recombinant human glutaryl-CoA dehydrogenase (rhGCDH) for treatment of glutaric aciduria and a method for treatment of glutaric aciduria, including a step of administering the pharmaceutical composition.

### BACKGROUND

Glutaric aciduria or glutaric acidemia is a disorder in which the body is unable to completely breakdown the amino acids lysine, hydroxylysine, and tryptophan and accumulates excessive levels of their intermediate breakdown products, for example, glutaric acid or 3-hydroxyglutaric acid, therein. Glutaric aciduria is an autosomal recessive inborn error of metabolism caused by a deficiency of the enzyme glutaryl-CoA dehydrogenase and is divided into glutaric aciduria type 1 (GA-1) and glutaric aciduria type 2 (GA-2).

In most babies with glutaric aciduria, macrocephaly is found. In addition, the babies may exhibit various degrees of neurological symptoms, for example, whining, opisthotonus, hypotonia, dystonia, chorea, convulsion, developmental delay, etc. Many babies with glutaric aciduria are born with unusually large heads or exhibit macrocephaly immediately after birth. Usually, such babies increase in head circumference within several weeks to months after birth. However, some babies may normally develop to an age of two and may undergo late-onset after the age of six.

Glutaric aciduria type 1 (GA-1) is known to be caused by defect or deficiency of mitochondrial glutaryl-CoA dehydrogenase (GCDH) while mutations in the ETFA, ETFB, and ETFDH genes cause glutaric aciduria type 2 (GA-2).

Glutaric aciduria is a fatal disease that can lead to death within 10 years of age if not treated, but patients with mild symptoms, who have been diagnosed, treated, and managed at early stage, can normally develop through protein restriction and supplemental therapy with carnitine and riboflavin. However, early diagnosis, treatment, and management are essential because the therapeutic effect is very low for an already damaged brain. The treatment includes emergency treatment, dietary therapy, substitution therapy with arginine, carnitine, clofibrate, and/or riboflavin, neuropharmacological therapy, and multidisciplinary supportive therapy.

Children with glutaric aciduria should be on a low-protein or lysine-free diet, and tryptophan should also be reduced in the diet. However, a diet without tryptophan can cause serious side effects, so that the children should be supplied with a low-tryptophan diet. Diet and supplementation with carnitine do not improve the main clinical symptoms in children with such neurological symptoms, but are known to control disease progression.

For a disease caused by a single enzyme defect or deficiency, enzyme replacement therapy, in which the deficient enzyme is administered into the body, can be contemplated. However, treatments for many diseases that cause brain damage often fail to prevent brain damage unless they are administered directly into the ventricle. This is due to the blood-brain barrier (BBB), which restricts the exchange of substances between the brain parenchyma and blood, and most proteins and components with high molecular weight do not pass through the barrier.

Among mucopolysaccharidosis, Hunter's syndrome, Hurler's syndrome and Sanfilippo syndrome are diseases that primarily affect the brain and in which the body does not properly break down precursors due to deficiency of specific enzymes, like glutaric aciduria type 1. In this case, injection of the deficient enzyme into the blood vessel did not succeed in passing through the blood brain barrier, with the consequent failure of preventing brain damage and brain function from deteriorating, although improving only the soft tissues (Lamp, C. et al., J. Inherit. Metab. Dis., 2014; Bradley, L.A. et al., Genet. Med., 2017; Sohn, YB. et al., Orphanet J. Rare Dis., 2013; Muenzer, J. et al., Genet. Metab., 2007; Muenzer, J. et al., Genet. Med., 2006; Muenzer, J. et al., Genet. Med., 2011). The only way to prevent brain function from deteriorating is to administer deficiency enzymes directly into the ventricles, which require insertion of a certain device (Ommaya device) through brain surgery and direct administration of the deficiency enzymes to break down the precursors accumulated in the brain (Sohn, YB. et al., J. Inherit. Metab. Dis., 2018; Giugliani, R. et al., Expert Opin. Orphan Drugs, 2018). This type of diseases includes GM1-gangliosidosis and Krabbe disease in addition to the aforementioned diseases.

Glutaric aciduria has a poor prognosis even if it is controlled by extreme dietary restrictions, and a therapeutic agent that can effectively control the disease has not yet been developed. Thus, the development of a therapeutic agent and method for effective treatment of it is required, and there is a need to develop a therapeutic agent and a treatment method that can bring about a therapeutic effect by intravenous or subcutaneous administration without the hassle of direct administration into the ventricle as in the conventional diseases causing brain damage.

### [Related Art Document]

(Patent Literature 1) International Patent Number WO2013133691 A1
(Patent Literature 2) International Patent Number WO2013133711 A1
(Non- Patent Literature 1) Kolker et al., "Diagnosis and management of glutaric aciduria type I - revised recommendations"; Journal of Inherited Metabolic Disease, Published online Mar 23, 2011; 34(3): pp 677-694.

### SUMMARY

The present disclosure is to provide a therapeutic agent capable of effective treating glutaric aciduria and an administration method therefor.

Leading to the present disclosure, intensive and thorough research, conducted by the present inventors into the development of a therapeutic agent capable of effectively treating glutaric aciduria, resulted in developing a pharmaceutical composition containing recombinant human glutaryl-CoA dehydrogenase (rhGCDH) for treatment of glutaric aciduria and a method for administering the pharmaceutical composition *in vivo* (intravenous (*i.v.*) and subcutaneous (*s.c*.) and finding that application of the pharmaceutical composition and the administration method brings about a significant reduction in glutaric acid (GA) in mouse animal models.

An aspect of the present disclosure is to provide a pharmaceutical composition containing recombinant human glutaryl-CoA dehydrogenase (rhGCDH) for treatment of glutaric aciduria.

The composition of the present disclosure may be a pharmaceutical composition for treatment of glutaric aciduria, designed to be administered *in vivo,* especially intravenously or subcutaneously.

Another aspect of the present disclosure is to provide a method for treatment of glutaric aciduria, comprising a step of administering the pharmaceutical composition intravenously or subcutaneously.

The pharmaceutical composition containing recombinant human glutaryl-CoA dehydrogenase (rhGCDH) for treatment of glutaric aciduria and the method for administering the pharmaceutical composition *in vivo* (intravenously (*i.v*.) and subcutaneously (*s.c*.) according to aspects of the present disclosure allow for the provision of a novel therapy for glutaric aciduria and exhibit excellent effects even upon *in vivo* administration remarkably easier than intraventicular administration, thus finding effective applications for controlling and treating glutaric aciduria.

### BRIEF DESCRIPTION OF THE DRAWINGS

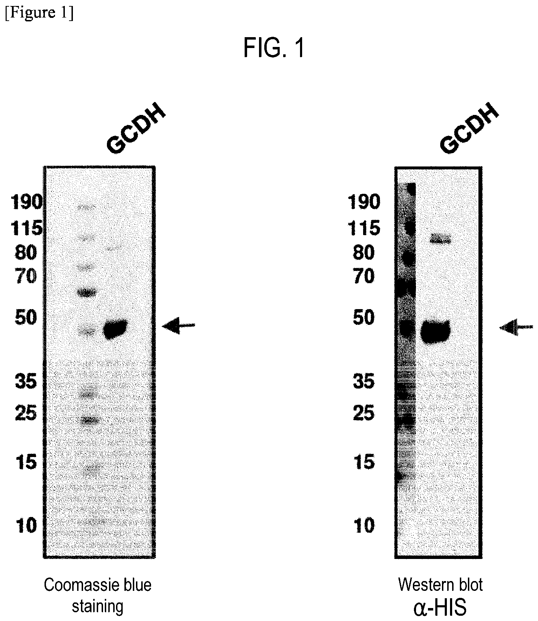

FIG. 1 shows purity of isolated recombinant human glutaryl-CoA dehydrogenase (rhGCDH) as measured by Coomassie Blue staining (panel A) and Western blot (panel B).

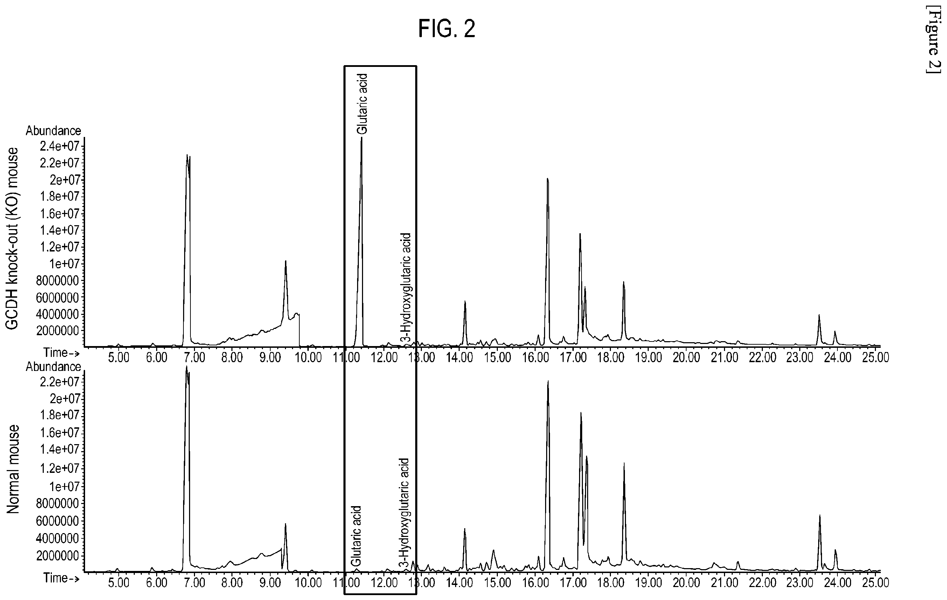

FIG. 2 shows GC-MSD (gas chromatography mass spectrometry) spectra of urine glutaric acid (GA) and 3-hydroxyglutaric acid (3-OHGA) levels in normal mice and GCDH KO mice, indicating that the established GCDH KO mice are useful as animal models of glutaric aciduria. In red box of the spectra, the left peak accounts for glutaric acid and the right peak for hydroxyglutaric acid.

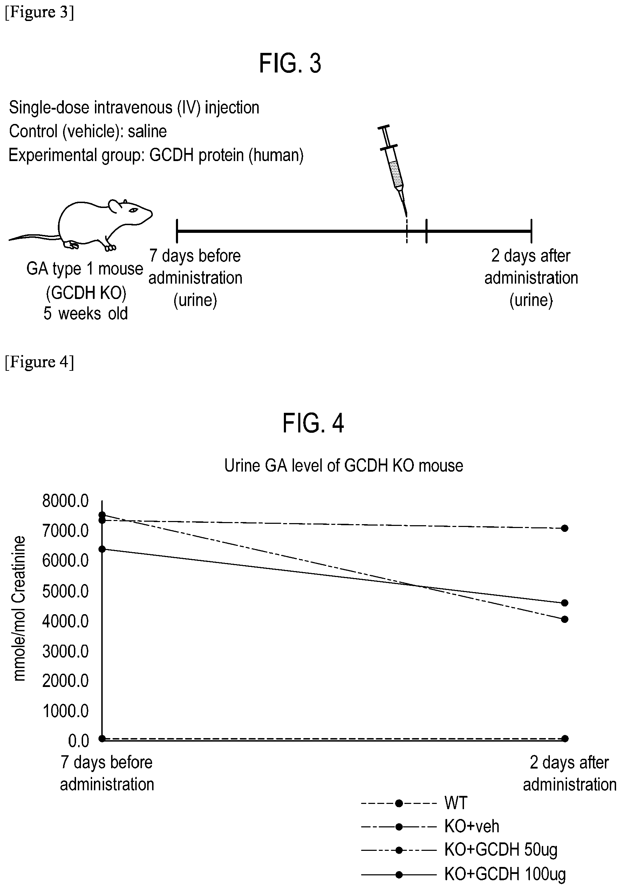

FIG. 3 is a scheme of a primary animal experiment for GCDH KO mice, which are animal models of glutaric aciduria, wherein urine samples are taken one week before and two days after the administration of recombinant human GCDH at a single dose of 50 or 100 µg via the tail vein and measured for glutaric acid level by LC-MS.
FIG. 4 is a plot of glutaric acid levels in urine samples taken one week before and two days after the administration of recombinant human GCDH to the glutaric aciduria animal models GCDH KO mice in a primary animal experiment, as measured by LC-MS.

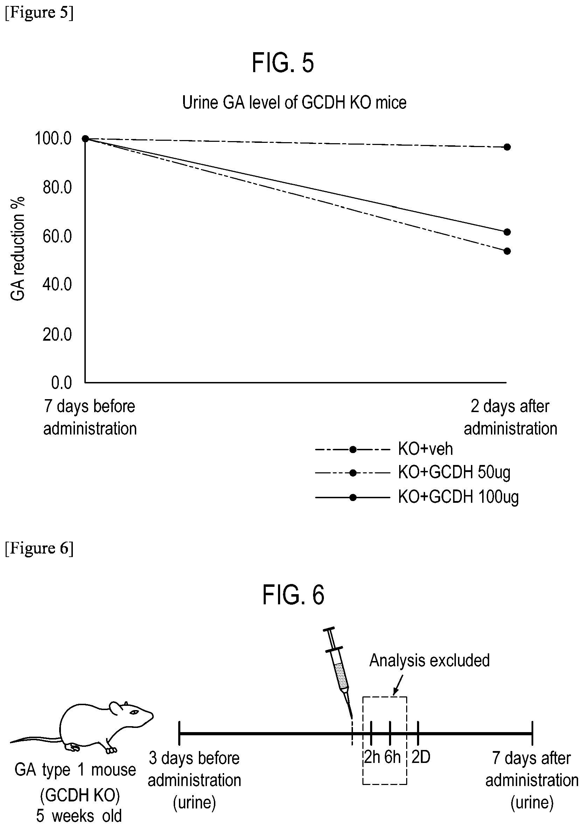

FIG. 5 is a plot of glutaric acid levels in urine samples taken one week before and two days after the administration of recombinant human GCDH to the glutaric aciduria animal models GCDH KO mice in a primary animal experiment, as measured by LC-MS, as calculated as percentages on the basis of the level before administration (100%).
FIG. 6 is a scheme of a secondary animal experiment for GCDH KO mice, which are animal models of glutaric aciduria, wherein urine samples are taken three days before and two days and one week after the administration of recombinant human GCDH at a single dose of 50 or 100 µg via the tail vein and at a single dose of 50, 150, or 250 µg via a subcutaneous route and measured for glutaric acid level by LC-MS.

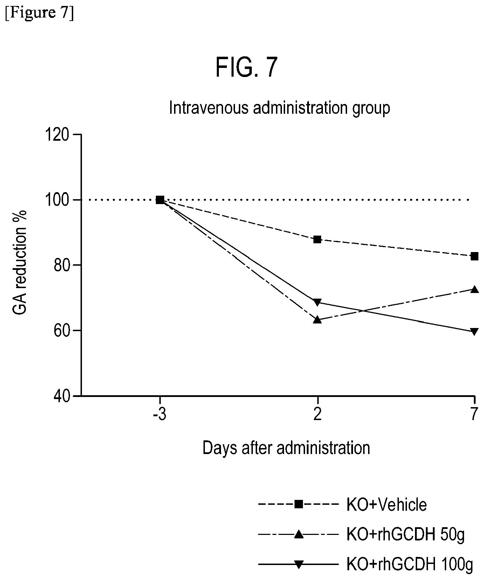

FIG. 7 is a plot of glutaric acid levels in urine samples taken three days before and two days and one week after the administration of recombinant human GCDH to the glutaric aciduria animal models GCDH KO mice via the tail vein in a secondary animal experiment, as expressed as percentages on the basis of the level before administration (100%).

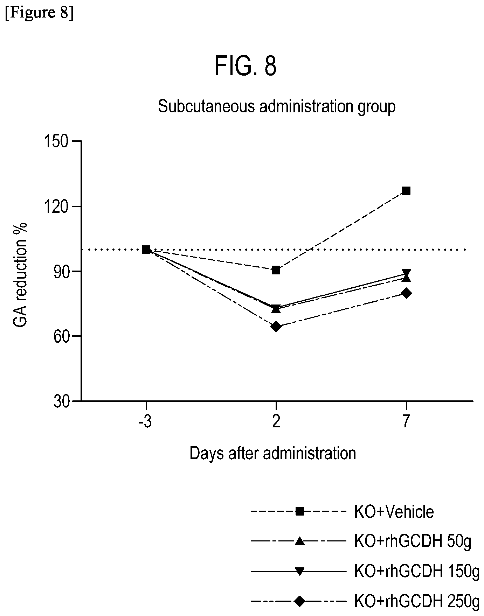

FIG. 8 is a plot of glutaric acid levels in urine samples taken three days before and two days and one week after the administration of recombinant human GCDH to the glutaric aciduria animal models GCDH KO mice via a subcutaneous route in a secondary animal experiment, as calculated as percentages on the basis of the level before administration (100%).

### DETAILED DESCRIPTION

Embodiments of the present disclosure are illustrated for describing the technical spirit of the present disclosure. The scope of the claims according to the present disclosure is not limited to the embodiments described below or to the detailed descriptions of these embodiments.

In addition, those skilled in the art to which the present disclosure belongs will recognize, or be able to ascertain many equivalents to the specific embodiments of the invention described herein by using no more than routine experimentation. Furthermore, these equivalents should be interpreted to fall within the present invention.

All technical or scientific terms used herein have meanings that are generally understood by a person having ordinary knowledge in the art to which the present disclosure pertains, unless otherwise specified. The terms used herein are selected for only more clear illustration of the present disclosure, and are not intended to limit the scope of claims in accordance with the present disclosure.

The expressions "include(contain/comprise)", "provided with", "have" and the like used herein should be understood as open-ended terms connoting the possibility of inclusion of other embodiments, unless otherwise mentioned in a phrase or sentence including the expressions.

In understanding the scope of the present disclosure, the term "consisting of" said elements, and its derivatives, as used herein, are intended to be close-ended terms that specify the presence of stated elements, and also exclude the presence of other unstated elements.

A singular expression can include meanings of plurality, unless otherwise mentioned, and the same is applied to a singular expression stated in the claims.

In an aspect of the present disclosure, the term "composition containing recombinant human glutaryl-CoA dehydrogenase" (rhGCDH) refers to a composition containing a protein product obtained by expressing a glutaryl-CoA dehydrogenase through a genetic recombination technique. The pharmaceutical composition of the present disclosure includes the rhGCDH of the present disclosure without limitations. In an embodiment of the present disclosure, the pharmaceutical composition of the present disclosure contains a pharmaceutically effective amount of the rhGCDH of the present disclosure and may further include a pharmaceutically acceptable carrier.

As used herein, the term "glutaric aciduria" means glutaric aciduria type 1, which is a disorder caused as the body is unable to completely breakdown the amino acids lysine, hydroxylysine, and tryptophan and accumulates excessive levels of their intermediate breakdown products therein, thus suffering from various symptoms due to defect or deficiency of GCDH. Most babies with glutaric aciduria exhibit macrocephaly and various degrees of neurological symptoms (whining, opisthotonus, hypotonia, dystonia, chorea, convulsion, developmental delay, etc.).

Herein, the glutaryl-CoA dehydrogenase (GCDH) is an enzyme that catalyzes the oxidative decarboxylation of glutaryl-CoA to crotonyl-CoA in the degradative pathway of lysine or tryptophan metabolism and may be GCDH classified as EC 1.3.99.7. on the basis of the Enzyme Nomenclature Committee of the International Union of Biochemistry and Molecular Biology. Herein, the GCDH may include the amino acid sequence of SEQ ID NO: 1, which is based on the Homo sapiens GCDH sequence of NCBI accession No. NP_000150.1 (https://www.ncbi.nlm.nih.gov/protein/NP_000150.1).

Alternatively, the amino acid sequence may include an amino acid sequence that has a homology of about 80% or more, particularly about 90% or more, more particularly about 95% or more, even more particularly about 97% or more, and most particularly about 99% or more with the amino acid sequence of SEQ ID NO: 1. If it accounts for a protein that having an activity substantially identical or corresponding to that of the protein including the amino acid sequence of SEQ ID NO: 1, any amino acid sequence with such homology may be included without limitations. In addition, it is obvious that an amino acid sequence derived by deletion, modification, substitution, or addition from the amino acid sequence having such homology also fall within the scope of the present disclosure. For instance, a part of the amino acid sequence may undergo deletion, modification, substitution, or addition in order to optimize the expression and acquiring process. In detail, the signal amino acid sequence of SEQ ID NO: 2 may be further included, but with no limitations thereto. In the present disclosure, the signal amino acid sequence of SEQ ID NO: 2 was added to the N terminus of the amino acid sequence of SEQ ID NO: 1 to enhance the expression and acquirement of GCDH in CHO cells.

The term "homology," used in herein, refers to the degree of identity of bases or amino acid residues between sequences after aligning both amino acid sequences or base sequences of a gene encoding a polypeptide in a specific comparison region to be matched with each other as much as possible. When the homology is sufficiently high, expression products of the corresponding gene may have the same or similar activity. The percentage of the sequence identity can be determined using a known sequence comparison program (e.g., BLAST (NCBI), etc.).

As used herein, the term "about" is intended to include an acceptable error range in view of manufacturing process for a particular value or a slight numerical modification to the particular value within the scope of the technical spirit of the present disclosure. For instance, the term "about" means a range of ±10% of a given value, ±5% of a given value in an aspect, and ±2% of a given value in another aspect. For the field of this disclosure, this level of approximation is appropriate unless the value is specifically stated require a tighter range.

In the present disclosure, proteins can be expressed and produced through methods known in the art to which the present disclosure belongs. Herein, the host cells suitable for expressing and producing the protein include higher eukaryotic cells disclosed in the present application. Proliferation of vertebrate cells in culture media becomes a general technique. Examples of useful vertebrate host cells include monkey kidney cells (CV1), SV40-transformed monkey kidney cell line CV1 (COS-7), human embryonic kidney cells (293 cells), baby hamster kidney cells (BHK), Chinese hamster ovarian cells (CHO), mouse sertoli cells (TM4), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor (MMT 060562), TRI cells, MRC 5 cells, and FS4 cells, but are not limited thereto.

The host cells used to produce the protein of the present disclosure may be cultured in a variety of media. Commercially available media such as Ham's F10, Minimal Essential Medium (MEM), RPMI-1640, and Dulbecco's Modified Eagle's Medium (DMEM), and any medium used in the art to which the present disclosure belongs may be used for culturing the host cells. Any of these media can be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™}), trace elements, and glucose or an equivalent energy source, and the like. Any other necessary/auxiliary supplements can also be included at appropriate concentrations that would be known to those skilled in the art. The culture conditions, such as temperature, pH, CO₂ concentration, and the like, are those previously used with the host cell selected for the protein expression, and will be apparent to the ordinarily skilled artisan.

The protein of the present disclosure can be purified using any protein purification method known in the art to which the present disclosure belongs. By way of example, various known techniques for protein purification such as fraction by hydrophobic interaction or immune affinity or on an ion-exchange column, ethanol precipitation, reverse-phase HPLC, chromatography on silica, chromatography on an anion or cation exchange resin, chromatofocusing, SDS-PAGE, ammonium sulfate precipitation, gel filtration, or genetic engineering technology (tag, etc.) are also available. Any combination of the mutiple purification methods may be employed in order to increase the purity of the protein.

In the present disclosure, the protein purified through the method may have a purity at a high level enough to allow for administration to animals, especially humans for experimental, clinical, and therapeutic purposes.

In the present disclosure, the recombinant human GCDH may be contained at a concentration of about 0.001 to about 500 mg/ml, but with no limitations thereto.

The pharmaceutical composition containing the recombinant human GCDH according to the present disclosure will be formulated and dosed in a fashion consistent with medical practice, taking into account the disease to be treated, specific animals to be treated, clinical condition of individual subjects, causes of disease, the site of delivery, the method of administration, the scheduling of administration, and other factors known to practitioners. The formulation in the present disclosure may include liquid formulations, or lyophilized powder, and so on. The pharmaceutical composition of the present disclosure can be formulated into dosage forms containable in a container, such as an ampoule, a vial, a bottle, a cartridge, a reservoir, Lyo-Ject, or a pre-filled syringe and may be prepared into single-dose forms or multi-dose forms.

The "pharmaceutically effective amount," "dose," "therapeutically effective amount," or "effective dose" of the recombinant human GCDH will be determined in consideration of the above factors and may be a minimum amount necessary for preventing, alleviating, or treating a specific disease. The "pharmaceutically effective amount," "dose," "therapeutically effective amount," or "effective dose" of the recombinant human GCDH according to the present disclosure may be, for example, about 0.0001mg/kg to about 100 mg/kg per dose. Accordingly, the composition of the present disclosure may contain the recombinant human GCDH in an amount of about 0.001 to about 1000 mg as a single dose. The pharmaceutical composition of the present disclosure may be periodically administered once a day, three times a week, twice a week, once a week, three times a month, twice a month, or once a month by the judgment of experienced practitioners and may be administered aperiodically in situations such as acute progression of a disease, but with no limitations thereto.

On the other hand, in the present disclosure, the pharmaceutical composition may be intended to be administered to a patient in parallel with an existing glutaric aciduria therapy. In detail, the pharmaceutical composition of the present disclosure may be administered to patients in combination with typical glutaric aciduria therapies, such as a therapy for reducing lysine-oxidation and increasing physiologic detoxification of glutaryl-CoA, supplementation therapy with arginine, carnitine, clofibrate, and/or riboflavin, dietary control such as low-protein diet or low-lysine/lysine-free diet. More specifically, the pharmaceutical composition of the present disclosure may be administered to a patient in parallel with the routinely progressed diet control.

The pharmaceutical composition in the present disclosure may be prepared by mixing the recombinant human GCDH having a desired degree of purity with a pharmaceutically or physiologically acceptable carrier, excipient, or stabilizer according to standard methods known in the art. Acceptable carriers may include saline or buffers such as phosphate, citrate, and other organic acids; antioxidants, such as ascorbic acid; low molecular weight (less than about 10 residues) polypeptides; proteins, such as serum albumin, gelatin, or immunoglobulins; hydrophilic polymers, such as polyvinylpyrrolidone; amino acids, such as glycine, glutamine, asparagine, arginine, or lysine; monosaccharides, disaccharides, and other carbohydrates, such as glucose, mannose, or dextrins; chelating agents, such as EDTA; sugar alcohols, such as mannitol or sorbitol; salt-forming counter-ions, such as sodium; and/or non-ionic surfactants, such as TWEEN^{™}, PLURONICS^{™}, or PEG.

The pharmaceutical composition of the present disclosure may contain a pharmaceutically acceptable salt at about physiological concentrations. Optionally, the formulations of the present disclosure may contain a pharmaceutically acceptable preservative. Specifically, the preservative concentration ranges from 0.1 to 2.0% (typically v/v). The preservatives include those known in the pharmaceutical arts. Specifically, benzyl alcohol, phenol, m-cresol, methylparaben, propylparaben, or a combination thereof may be employed. The pharmaceutical composition of the present disclosure may include a pharmaceutically acceptable surfactant at a concentration of 0.005 to 0.02%.

In the present disclosure, the pharmaceutical composition may further contain one or more active compounds necessary for the treatment of glutaric aciduria, specifically, an active compound having complementary activity that does not adversely affect each other with the recombinant human GCDH. The compound may be contained in the composition in an amount effective to achieve the intended purpose.

Provided according to an aspect of the present disclosure is a method for treating glutaric aciduria, including a step of administering the recombinant human GCDH-containing pharmaceutical composition. As use herein, the terms "recombinant human GCDH," "pharmaceutical composition containing recombinant human GCDH," and "glutaric aciduria" are as defined above.

The pharmaceutical composition containing recombinant human GCDH according to the present disclosure may be administered to humans or animal subjects via intravenous, intramuscular, intraperitoneal, intracerobrospinal, subcutaneous, intra-articular, intrasynovial, intrathecal, oral, topical, or inhalation routes.

As demonstrated in the following Examples, the pharmaceutical composition comprising recombinant human GCDH of the present disclosure is more preferably administered by intravenous or subcutaneous injection because the intravenous or subcutaneous route leads to excellent effects.

Hereinafter, the constituents and effects of the present disclosure will be more specifically described by referring to Examples and Test Examples. However, they are set forth only to illustrate the present disclosure to support understanding it, but not construed to limit the scope and range of the present disclosure.

### EXAMPLES

### EXAMPLE 1. Preparation and Purification of Recombinant Human Glutaryl-CoA Dehydrogenase (GCDH)

For use in producing recombinant human glutaryl-CoA dehydrogenase (GCDH), a plasmid optimized to express GCDH in CHO cells (*Cricetulus griseus*; Chinese Hamster cells) was constructed from the backbone of pcDNA3.4-TOPO. Accordingly, an expression plasmid for expressing GCDH protein including the amino acid sequence of SEQ ID NO: 1 was constructed (p18AFUOQC_GCDH_pcDNA3.4-TOPO). In this Example, the amino acid sequence of SEQ ID NO: 2, which functions as a signal sequence, was added to the N terminal of GCDH protein so that the plasmid could be optimally expressed and secreted.

The p18AFUOQC plasmid thus constructed was transformed into CHO cells (Expi^{™} CHO cells) which were than cultured at 37°C for 5 days in an 8% CO₂ atmosphere to express the protein.

After the cells were harvested, purification was made of the protein with the aid of an immunoaffinity column (HisTrap column). Purity of the protein was identified by Coomassie Blue staining and Western blotting (FIG. 1). The protein thus obtained had a concentration of 7.06 mg/ml.

### EXAMPLE 2. Glutaric Aciduria Animal Model

GCDH KO mouse strains are commercially available as mouse models of glutaric aciduria (e.g., Cyagen, strain name: KO - C57BL/6J-Gcdh^{em1cyagen}; Conditional KO -C57BL/6J-Gcdh^{cm1.1cyagcn}). On the other hand, GCDH KO mice were generated using CRISPR (Clustered Regularly Interspaced Short Palindromic Repeats) gene scissors technology by the present inventors in this disclosure. KO mice were generated by targeting the exon 2 of mouse GCDH. Macrogen Inc. was commissioned a specific generation. Genotyping was performed through a T7 endonuclease I reaction to examine whether GCDH in the generated mouse model of glutaric aciduria (GCDH KO mouse) was knocked out (Knock-out; KO). KO mice identified by genotyping were primarily selected (Table 1).

**TABLE 1**

| GCDH | No. of mice | Mouse No. (F0 #) |
|---|---|---|
| T7E1 analysis | 21 | 1 ∼ 21 |
| T7E1 positive | 21 | 1 ∼ 21 |
| WT mix T7E 1 (homo mutant predicted) | 4 | 4, 9, 13, 20 |
| Mass insertion/deletion (predicted) | 6 | 4, 5, 11, 14, 17, 18 |
| Total mutant | 21 | 1 ∼ 21 |
| Mutation efficiency (%) | (21/21) 100% | |
| Homo KO mutant generation efficiency (%) | (4/21) 19% | |

Specifically, the T7 endonuclease reaction was carried out to examine whether 21 F0 mutation-induced mice were mutated. As a result, mutation was induced in all of 21 mice. In particular, F0 #4, #9, #13, and #20 were considered to be homo mutants in which the KO mutation occurred on both of the alleles. Among them, F0 #4 was selected, maintained, and bred, and then used for subsequent experiments.

The GCDH KO mice thus obtained were identified to be an animal model of glutaric aciduria as follows. Specifically, normal mice and GCDH KO mice were measured for urine levels of glutaric acid (GA) and 3-hydroxyglutaric acid (3-OHGA) and blood levels of glutarylcarnitine (C5DC).

GA and 3-OHGA levels were determined by analyzing organic acids in urine through GC-MS while C5DC levels were obtained through analysis for dried-blood spot. The results are described in Table 2 below and depicted in FIG. 2.

**TABLE 2**

| | Dried-blood spot (DBS; µM) | Urine (mmol/mol Crea.) | |
|---|---|---|---|
| Average | C5DC | GA | 3-OHGA |
| Normal mouse 1 | 0.055 | 21.55 | 17.1 |
| Normal mouse 2 | | | |
| GCDH KO mouse 1 | 0.145 | 3206.8 | 20.8 |
| GCDH KO mouse 2 | | | |

As can be seen from the above data, the generated GCDH KO mice were remarkably high in C5DC and glutaric acid and the like, and thus exhibited very similar symptoms to those of glutaric aciduria type 1 patients, demonstrating the availability thereof as an animal model of GA1.

### EXAMPLE 3. Administration of Recombinant Human Glutaryl-CoA Dehydrogenase to Animal Model of Glutaric Aciduria Type 1

The recombinant human glutaryl-CoA dehydrogenase (GCDH) prepared in Example 1 was administered to the animal model of glutaric aciduria type 1 generated/established in Example 2, and the therapeutic effect was confirmed twice.

### Example 3-1. Primary in vivo assay

A single dose of the recombinant human GCDH was administered to 5-week-old GCDH KO mice via the tail vein. Specifically, rhGCDH was administered at a single dose of 50 or 100 µg, followed by LC-MS/MS analysis for urine glutaric acid (GA) level. GA levels were measured in urine collected from each experimental group one week before administration of rhGCDH and two days after administration. A scheme of the primary animal experiment is shown in FIG. 4 and each experimental group and the numbers of subjects of them are listed in Table 3, below.

**TABLE 3**

| Mouse genotype | Composition administered | No. of subjects |
|---|---|---|
| Wild-type (WT) | Vehicle | 2 |
| GCDH KO | Vehicle | 3 |
| GCDH KO | rhGCDH 50 µg | 4 |
| GCDH KO | rhGCDH 100 µg | 3 |

The glutaric acid levels in urine taken in the above-mentioned manner are given in Table 4, below, as measured by LC-MS/MS analysis. Since LC-MS/MS analysis requires a large amount of urine, the urine samples of all subjects in each experimental group were integrated and analyzed as one sample.

**TABLE 4**

| Experimental group | GA (mmol/mol creatinine) | |
|---|---|---|
| | 1 week before administration | 2 days after administration |
| WT + Veh | 100.1 | 46.0 |
| GCDH KO + Veh | 7357.4 | 7097.2 |
| GCDH KO + rhGCDH 50 µg | 7484.0 | 4073.2 |
| GCDH KO + rhGCDH 100 µg | 6389.1 | 4599.9 |
| GA average of 4-week-old GCDH KO mice | 7420.7 | - |

As be seen in Table 4 and FIGS. 4 and 5, the wild-type mice maintained glutaric acid at a level of about 100 mmol/mol or less whereas GCDH KO mice exhibited a glutaric acid level of about 6400 to 7500 mmol/mol before treatment with rhGCDH. Administration of rhGCDH decreased the glutaric acid level by about 30-45 % irrespective of doses.

### EXAMPLE 3-2. Secondary in vivo experiment

A single dose of the recombinant human GCDH was administered to GCDH KO mice 5 to 6 weeks old via the tail vein (*i.v.*) or subcutaneous (*s.c*.) route. Specifically, rhGCDH was administered at a single dose of 50 or 100 µg through the tail vein route and at a single dose of 50, 150, and 250 µg subcutaneously. Generally, the subcutaneous dose has a bioavailability (BA) of 20% compared to that of intravenous administration (see Korean Patent No. 2017-0004814 A), so the dose was set to 1, 3, and 5 times compared to 50 µg for intravenous administration.

Urine glutaric acid (GA) levels were measured by LC-MS/MS. GA levels were measured in the urine taken from mice in each experimental group 3 days before rhGCDH administration and 2 days and 1 weeks after rhGCDH administration. A scheme of the secondary animal experiment is depicted in FIG. 6, and each experimental group and the numbers of subjects of them are listed in Tables 5 and 6, below.

**TABLE 5**

| *i*.*v*. Administered Group | | |
|---|---|---|
| Mouse genotype | Composition administered | No of subjects |
| Wild-type (WT) | Vehicle | 4 |
| GCDH KO | Vehicle | 4 |
| GCDH KO | rhGCDH 50 µg | 4 |
| GCDH KO | rhGCDH 100 µg | 4 |

**TABLE 6**

| *s.c*. Administered Group | | |
|---|---|---|
| Mouse genotype | Composition administered | No of subjects |
| Wild-type (WT) | Vehicle | 5 |
| GCDH KO | Vehicle | 5 |
| GCDH KO | rhGCDH 50 µg | 5 |
| GCDH KO | rhGCDH 150 µg | 5 |
| GCDH KO | rhGCDH 250 µg | 5 |

The glutaric acid levels in urine taken in the above-mentioned manner are given in Table 7, below, as measured by LC-MS/MS analysis. In the secondary experiment, the levels were measured for each mouse and then the average value of the measurements was calculated.

**TABLE 7**

| i.v. Administration | GA Reduction % (mean±SEM) | | |
|---|---|---|---|
| | Before administration | 2 days after administration | 7 days after administration |
| WT +Veh | - | - | - |
| GCDH KO+ Veh | 100 | 87.86±6.015 | 82.71±7.579 |
| GCDH KO+ rhGCDH 50 µg | 100 | 63.45±17.642 | 72.67±21.116 |
| GCDH KO+ rhGCDH 100 µg | 100 | 68.40±9.985 | 59.57±5.687 |

**TABLE 8**

| s.c. Administration | GA Reduction % | | |
|---|---|---|---|
| | Before | 2 days after | 7 days after |
| | administration | administration | administration |
| WT +Veh | - | - | - |
| GCDH KO+ Veh | 100 | 90.60±8.735 | 127.32±16.785 |
| GCDH KO+ rhGCDH 50 µg | 100 | 72.94±11.503 | 87.25±10.317 |
| GCDH KO+ rhGCDH 150 µg | 100 | 72.87±4.858 | 88.90±0 |
| GCDH KO+ rhGCDH 250 µg | 100 | 64.49±17.421 | 79.94±9.677 |

Wild-type mice showed no significant reduction in glutaric acid, with average glutaric acid levels maintained at or below about 100 mmol/mol (marked with "-"). In contrast, GCDH KO mice were measured to have glutaric acid at a level of about 3600 to 10000 mmol/mol on average before treatment with rhGCDH. Administration of rhGCDH decreased glutaric acid levels by about 30-40 % irrespective of doses.

Table 9 below compares the effects of intravenous administration and subcutaneous administration.

**TABLE 9**

| Experiment | | GA Reduction % | | |
|---|---|---|---|---|
| | | Before administration | 2 days after administration | 7 days after administration |
| GCDH KO + Veh | *i.v.* | 100 | 87.9 ± 6.015 | 82.7 ± 7.579 |
| | *s.c.* | 100 | 90.6 ± 8.735 | 127.3 ± 16.785 |
| GCDH KO + rhGCDH 50µg (①) | *i.v.* | 100 | 63.5 ± 17.64 | 72.7 ± 21.12 |
| GCDH KO + rhGCDH 50µg | *s.c.* | 100 | 73.0 ± 11.50 | 87.3 ± 10.32 |
| GCDH KO + rhGCDH 100µg | *i.v.* | 100 | 68.4 ± 9.99 | 59.6 ± 5.69 |
| GCDH KO + rhGCDH 150µg | *s.c.* | 100 | 72.9 ± 4.96 | 88.9 |
| GCDH KO + rhGCDH 250µg(②) | *s.c.* | 100 | 64.5 ± 17.42 | 79.9 ± 9.68 |

Considering that the bioavailability of subcutaneous administration is generally 20% of that of intravenous administration (*see* Korean Patent No. 2017-0004814 A), it was found that ① GCDH KO + rhGCDH 50 µg *i.v.* experimental group and ② GCDH KO + rhGCDH 250 µg *s.c.* experimental group showed similar GA reduction effects at 2 days after administration and 7 days after administration.

As can be seen from the above data, administration of GCDH to the glutaric aciduria animal model could control the glutaric acid level with significance. It can also be seen that the effect remained effective up to one week after administration.

Taken together, the data indicate that the pharmaceutical composition containing the recombinant human glutaryl-CoA dehydrogenase (rhGCDH) of the present disclosure, even if not directly administered to the ventricle, therapeutically significantly reduces glutaric aciduria and maintains the reduced level through common *in vivo* administration routes, thereby finding advantageous application in therapy for glutaric aciduria.

From the above descriptions, a person skilled in the art to which the present disclosure pertains will understand that the present disclosure can be practiced in other specific forms without changing its technical idea or essential features. In this regard, it is understood that the examples described above are exemplary and not limiting in all respects. The scope of the present disclosure is construed so as to include the meaning and scope of the claims and all modifications or alternatives derived from the present disclosure and its equivalent concepts.

## Claims

1. A pharmaceutical composition for treatment of glutaric aciduria, the pharmaceutical composition comprising recombinant human glutaryl-CoA dehydrogenase (rhGCDH).

2. The pharmaceutical composition of claim 1, wherein the rhGCDH comprises the amino acid sequence of SEQ ID NO: 1.

3. The pharmaceutical composition of claim 1, wherein the pharmaceutical composition is administered intravenously or subcutaneously.

4. The pharmaceutical composition of claim 1, wherein the glutaric aciduria is glutaric aciduria type 1.

5. The pharmaceutical composition of claim 1, wherein the pharmaceutical composition is administered once a week, or once or twice a month.

6. The pharmaceutical composition of claim 1, wherein administration of the pharmaceutical composition is conducted in combination with a dietary control therapy for glutaric aciduria.

7. The pharmaceutical composition of claim 6, wherein the dietary control therapy for glutaric aciduria is low-protein diet, a low-lysine diet, a lysine-free diet, an arginine, carnitine, clofibrate and/or riboflavin supplementation therapy, or a combination thereof.

8. A method for treatment of glutaric aciduria, the method comprising a step of administering a pharmaceutical composition comprising recombinant human glutaryl-CoA dehydrogenase (rhGCDH) for glutaric aciduria.

9. The method of claim 8, wherein the rhGCDH comprises the amino acid sequence of SEQ ID NO: 1.

10. The method of claim 8, wherein the pharmaceutical composition is administered intravenously or subcutaneously.

11. The method of claim 8, wherein the glutaric aciduria is glutaric aciduria type 1.

12. The method of claim 8, wherein the pharmaceutical composition is administered once a week, or once or twice a month.

13. The method of claim 8, wherein administration of the pharmaceutical composition is conducted in combination with a dietary control therapy for glutaric aciduria.

14. The method of claim 13, wherein the dietary control therapy for glutaric aciduria is low-protein diet, a low-lysine diet, a lysine-free diet, a supplementation therapy with arginine, carnitine, clofibrate and/or riboflavin, or a combination thereof.
